# FASCICULE DE BREVET EUROPEEN

(11) **EP 3 141 694 B1**
(45) Date de publication et mention de la délivrance du brevet: **14.03.2018**
(21) Numéro de dépôt: 16187467.2
(22) Date de dépôt: 06.09.2016
(51) Int. Cl.: E21B 41/00, E21B 47/01, E21B 47/06, E21B 49/08, G01N 33/24

(54) **SONDE DE MESURES PERMANENTES DANS LE SOL ET SOUS-SOL**
SONDE FÜR KONTINUIERLICHES MESSEN IN GRUND UND UNTERGRUND
CONTINUOUS MEASURING PROBE FOR GROUND AND UNDERGROUND

(30) Priorité: 08.09.2015 FR 1558326
(43) Date de publication de la demande: 15.03.2017
(73) Titulaire: IFP Energies nouvelles, 92500 Rueil-Malmaison (FR)
(72) Inventeur: ROUCHON, Virgile, 92210 SAINT-CLOUD (FR); LANGLOIS, Bernard, 78100 ST GERMAIN EN LAYE (FR); FERNE, Eric, 91270 VIGNEUX SUR SEINE (FR); FERNANDES-MARTO, Claudio, 78300 POISSY (FR); GARCIA, Bruno, 92500 RUEIL MALMAISON (FR); LOISY, Corinne, 33400 TALENCE (FR); CEREPI, Adrian, 33600 PESSAC (FR); LE ROUX, Olivier, 33600 PESSAC (FR); SZABO, Zsuzsanna, 92500 RUEIL MALMAISON (FR)

(56) Documents cités:
- EP-A1- 0 621 488
- WO-A1-95/08129
- WO-A1-2014/087061
- CH-A- 486 698

## Description

La présente invention concerne la surveillance de l'impact sur le sol et le sous-sol d'activités anthropiques impliquant ou générant des fluides, et notamment des gaz.

Parmi ces activités on peut citer le stockage de déchets (par exemple des déchets nucléaires), les réseaux de transport de fluides (« pipelines » en anglais), le stockage de fluides souterrains (carburants, gaz), la pollution accidentelle des sols, la pollution liée aux activités industrielles non accidentelles, la production de fluides géologiques (hydrocarbures, eaux profondes, géothermie).

Plus particulièrement, la présente invention peut concerner la surveillance de sites de stockage géologique d'un fluide, tel que le dioxyde de carbone (CO2) ou le méthane.

La surveillance environnementale de l'impact d'activités anthropiques sur le sol et le sous-sol nécessite de disposer d'outils permettant la réalisation de mesures représentatives, normalisées, et sur des durées relativement longues (année, décennies).

La mesure des échanges gazeux dans le sol et le sous-sol est un des aspects de la surveillance environnementale, qui concerne à la fois la performance des sols, mais aussi la migration de gaz possible au travers des sol et sous-sol. Dans le cadre de la mesure des échanges gazeux dans le sol et le sous-sol, il peut être souhaitable de disposer de dispositifs de mesure permettant de prendre en compte une chaîne de processus allant du prélèvement de signaux ou de matière, en passant par la mesure de paramètres significatifs, jusqu'au traitement de la mesure et l'analyse d'incertitudes. Afin d'assurer la fiabilité et la représentativité des mesures, il est souhaitable que la mise en oeuvre de cette chaîne de processus perturbe le moins possible l'environnement à caractériser. L'interface physique d'un dispositif de mesure est donc un élément critique pouvant concentrer à lui seul les risques de perturbation de l'environnement ainsi que la représentativité des informations recueillies.

Par ailleurs, il peut être intéressant de croiser les mesures des échanges gazeux avec d'autres types de mesures physiques afin, par interprétation croisée, d'affiner la compréhension des mouvements de fluides dans une formation souterraine. Idéalement, pour garantir une représentativité des différents paramètres mesurés et limiter la perturbation de l'environnement, ces mesures doivent être réalisées simultanément et au même emplacement.

Enfin, idéalement, un dispositif de mesure pour la surveillance d'une formation souterraine contenant un fluide doit pouvoir résider dans son environnement de mesure aussi longtemps que nécessaire, en lien continu ou non avec des moyens de d'analyse de ces mesures et/ou d'alimentation, éventuellement déportés.

### État de la technique

Les documents suivants seront cités au cours de la description :
Noemie Taquet, Jacques Pironon, Philippe De Donato, Herve Lucas, Odile Barres (2013). Efficiency of combined FTIR and Raman spectrometry for online quantification of soil gases: Application to the monitoring of carbon dioxide storage sites. International Journal of Greenhouse Gas Control, 12, 359-371.

Thomas M. Christy (1998). A Permeable Membrane Sensor for the Détection of Volatile Compounds in Soil. Symposium on the Application of Geophysics to Engineering and Environmental Problems 1998: pp. 65-72.

On connaît le document (Taquet et al., 2013) qui décrit un dispositif dédié à la collecte d'informations relatives aux gaz du sol, la collecte étant réalisée dans un (mini-)puits de forage. Plus précisément, ce document décrit une chambre de prélèvement de gaz constituée par la base d'un puits, isolée de l'atmosphère du puits par un coussin gonflable connu sous le terme de « packer » en anglais. Le gaz est extrait de la chambre par une pompe, traverse une série d'analyseurs et de capteurs, puis est réinjecté au niveau de la chambre de prélèvement via des tubes en acier inox. De plus, une mesure de température est effectuée au niveau de la chambre de prélèvement. Des équipements en surface permettent de faire des mesures de la composition du gaz, de la pression et du débit de gaz, ainsi que de maintenir la pression du packer. La chambre de prélèvement est équipée d'une membrane semi-perméable permettant d'admettre dans les tubes les espèces gazeuses, sans laisser pénétrer l'eau liquide. Ce système, de par l'installation et le maintien de la pression des packers, est difficile de mise en oeuvre. Par ailleurs, ce document ne décrit ni ne mentionne de caractéristiques permettant d'assurer la non perturbation de l'environnement de mesure, ni la résistance à la corrosion dans le temps.

On connaît également la société GEOPROBE qui développe des systèmes dédiés à l'échantillonnage des gaz du sol. Ces systèmes, réalisés en acier permettent de forer rapidement différents types de sol et de récupérer les gaz présents en fond de forage. Plus particulèrement, les sondes Geoprobe MIP (voir par exemple (Christy, 1998)) permettent de faire remonter le gaz du sol via la circulation d'un gaz vecteur, et de mesurer la conductivité électrique du sol. Ces sondes, fabriquées en acier et les mesures étant réalisées en cours de forage, sont donc destinées à des applications ponctuelles, et ne sont pas dédiées à une surveillance à long terme d'un site de stockage. De plus, ces sondes requièrent l'injection d'un gaz vecteur, autre que le gaz prélevé, et ne réinjectent pas le gaz prélevé, ce qui ne permet pas de garantir la non perturbation de l'environnement de mesure.

On connaît également le brevet CH 486698 A qui concerne un dispositif pour mesurer l'humidité du sol, à partir d'une mesure de la capacité électrique et de la porosité.

Ainsi, aucun des dispositifs et systèmes selon l'art antérieur ne garantit, tout à la fois, la non perturbation de l'environnement de mesure, la mesure de la teneur en gaz, la mesure de propriétés électriques des sols, et l'adaptation, par leur fonctionnement et leurs matériaux, à une surveillance à long terme d'une formation contenant un fluide.

La présente invention décrit quant à elle un dispositif souterrain pour la surveillance d'une formation souterraine contenant un fluide, permettant au moins le prélèvement et l'analyse du fluide présent dans la formation, ainsi que la mesure de propriétés électriques de la formation étudiée. La sonde selon l'invention comprend une cellule de mesure composée au moins de deux chambres : une première chambre dans laquelle sont réellement effectuées les mesures, et une deuxième chambre de protection des moyens de liaison entre les instruments de mesure et des moyens d'analyse de ces mesures. Les moyens d'analyse des mesures peuvent être déportés, en surface par exemple, et les moyens de liaison peuvent comprendre une gaine étanche et résistante à la corrosion.

Ainsi, le dispositif selon l'invention est adapté à une surveillance à long terme d'une formation souterraine contenant un fluide, telle qu'un site de stockage géologique de gaz.

### Le dispositif selon l'invention

Ainsi, la présente invention concerne un dispositif pour la surveillance d'une formation souterraine contenant un fluide, ledit dispositif comportant au moins une cellule de mesure, destinée à être disposée dans une cavité formée dans ladite formation souterraine, des moyens d'analyse destinés à être en surface, des moyens de liaison reliant ladite cellule de mesure auxdits moyens d'analyse, ladite cellule comprenant au moins une première chambre, une deuxième chambre étanche audit fluide, et au moins trois connecteurs internes reliant de manière étanche ladite première chambre à ladite deuxième chambre, lesdits moyens de liaison reliant de manière étanche ladite cellule de mesure auxdits
- une pluralité d'orifices permettant le passage dudit fluide dans ladite première chambre,
- au moins deux électrodes internes coopérant avec au moins deux desdits connecteurs internes, lesdites électrodes internes étant reliées auxdits moyens d'analyse via lesdits moyens de liaison, lesdits moyens de liaison coopérant avec lesdits connecteurs internes,
- des moyens de circulation de fluide, lesdits moyens de circulation de fluide étant reliés auxdits moyens d'analyse en coopérant avec au moins un desdits connecteurs et avec lesdits moyens de liaison.

Selon un mode de mise en oeuvre de l'invention, le dispositif peut comporter en outre au moins deux électrodes externes, deux connecteurs internes reliant lesdites deux chambres, deux connecteurs externes étanches placés sur au moins une paroi de ladite première chambre, ladite paroi étant en contact avec ladite formation, lesdites électrodes externes coopérant avec lesdits connecteurs externes, lesdites électrodes externes étant reliées auxdits moyens d'analyse via lesdits moyens de liaison, lesdits moyens de liaison coopérant avec lesdits deux connecteurs internes et lesdits deux connecteurs externes.

Selon un mode de mise en oeuvre de l'invention, lesdits moyens de liaison peuvent comporter des moyens d'alimentation électrique desdites électrodes, et les moyens de protection étanches comporte une gaine étanche protégeant au moins lesdits moyens de circulation et lesdits moyens d'alimentation électrique.

Avantageusement, ladite première chambre peut être remplie par un matériau poreux et perméable pour lequel on connaît des propriétés pétrophysiques et électriques.

Préférentiellement ; lesdites propriétés pétrophysiques peuvent être au moins la porosité et la perméabilité, et lesdites propriétés électriques peuvent être au moins la conductivité électrique.

Selon un mode de mise en oeuvre de l'invention, lesdits moyens de liaison, lesdites chambres, et lesdits connecteurs peuvent être fabriqués en PTFE.

Selon un mode de mise en oeuvre de l'invention, lesdits moyens d'analyse peuvent au moins comporter un analyseur de fluide et/ou un résistivimètre.

Selon un mode de mise en oeuvre de l'invention, lesdits moyens de circulation de fluide peuvent comprendre au moins un tuyau, un système d'aspiration de fluide, et un système de reflux de fluide.

Avantageusement, lesdits moyens de circulation de fluide peuvent comprendre au moins deux tuyaux, un système d'aspiration de fluide, et un système de reflux de fluide.

De plus, l'invention concerne une utilisation du dispositif selon l'invention pour la surveillance d'un site de stockage géologique d'un gaz, tel que du CO₂ ou du méthane.

Préférentiellement, une étape d'étalonnage peut être réalisée préalablement à l'injection de gaz dans ledit site de stockage géologique lors d'une utilisation du dispositif selon l'invention pour la surveillance d'un site de stockage géologique d'un gaz.

En outre, l'invention concerne un procédé de surveillance d'une formation souterraine contenant un fluide, dans lequel on utilise au moins un dispositif pour la surveillance d'une formation souterraine contenant un fluide selon l'invention, et dans lequel on réalise au moins les étapes suivantes :
∘ on fore une cavité destinée à recevoir ladite cellule de mesure dudit dispositif ;
∘ on connecte ladite cellule de mesure auxdits moyens de liaison dudit dispositif ;
∘ on installe ladite cellule de mesure au sein de ladite cavité ainsi formée, et on installe lesdits moyens d'analyse dudit dispositif en surface ;
∘ on connecte lesdits moyens d'analyse auxdits moyens de liaison dudit dispositif ;
∘ on réalise des mesures au moyen de ladite cellule de mesure et on analyse lesdites mesures au moyen desdits moyens d'analyse.

D'autres caractéristiques et avantages du procédé selon l'invention, apparaîtront à la lecture de la description ci-après d'exemples non limitatifs de réalisations, en se référant aux figures annexées et décrites ci-après.

### Présentation succincte des figures

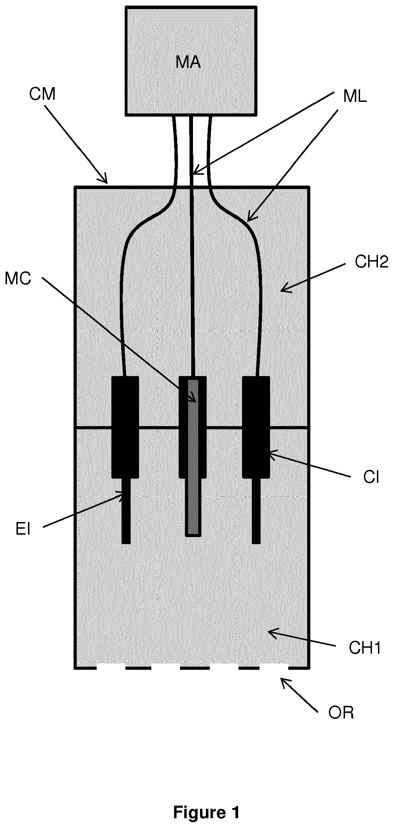

Les Figures 1 à 5 illustrent schématiquement et en coupe, différents modes de réalisation du dispositif pour la surveillance d'une formation souterraine contenant un fluide selon l'invention.

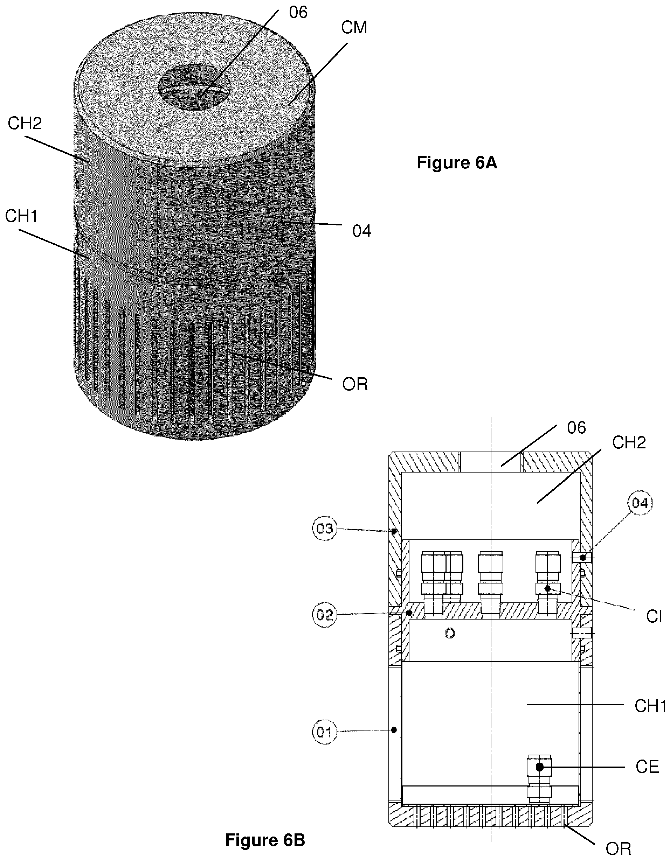

La Figure 6A illustre une vue externe d'un mode de réalisation de la cellule de mesure du dispositif pour la surveillance d'une formation souterraine contenant un fluide selon l'invention. La Figure 6B correspond à une section verticale au travers de la cellule de mesure du dispositif selon l'invention représentée en Figure 6A.

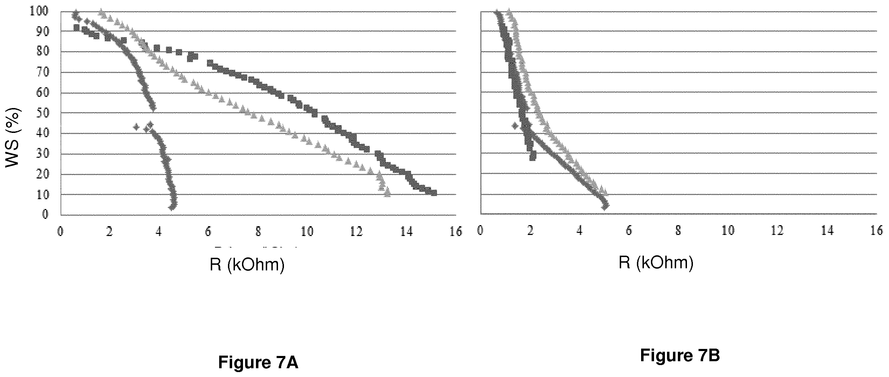

La Figure 7A (respectivement la Figure 7B) présente l'évolution de la résistivité électrique mesurée par des électrodes internes (respectivement par des électrodes externes) en fonction de la saturation en eau, pour trois différentes configurations d'assemblage de la cellule de mesure du dispositif selon l'invention.

### Description détaillée du dispositif

L'invention concerne un dispositif pour la surveillance d'une formation souterraine contenant un fluide. Le fluide peut être du gaz, tel que du CO₂ ou du méthane, ou bien un liquide tel qu'une phase liquide d'hydrocarbures. Le fluide peut avoir été, non limitativement, volontairement stocké dans la formation étudiée (par exemple dans le cas d'un stockage géologique de CO₂) ou bien résulter d'une dégradation de produits stockés dans la formation étudiée (par exemple dans le cas d'un stockage géologique de déchets), ou encore être un fluide naturel piégé géologiquement dans une formation du sous-sol.

Le dispositif selon l'invention comporte une cellule de mesure, destinée à être disposée dans une cavité formée dans la formation souterraine considérée. La cavité peut avoir été formée par un forage par exemple, à la taille de la cellule de mesure, de sorte à ce que la cellule soit en contact direct avec la formation. Selon l'invention, la cellule de mesure est constituée d'au moins une première chambre et une deuxième chambre. Dans la première chambre sont réellement effectuées les mesures, via des instruments de mesure, et la deuxième chambre sert de protection à des moyens de liaison reliant lesdits instruments de mesure à des moyens d'analyse de ces mesures. Les moyens d'analyse des mesures peuvent être déportés, en surface par exemple. Selon l'invention, les moyens de liaison relient de manière étanche la cellule de mesure aux moyens d'analyse et les moyens de liaison comportent des moyens de protection étanches.

De plus, au moins trois connecteurs internes relient de manière étanche ladite première chambre à ladite deuxième chambre, ces connecteurs permettent le passage de la mesure.

En outre, ladite première chambre comporte au moins :
- une pluralité d'orifices permettant le passage dudit fluide dans ladite première chambre,
- au moins deux électrodes internes coopérant avec au moins deux desdits connecteurs internes, lesdites électrodes internes étant reliées auxdits moyens d'analyse via lesdits moyens de liaison, lesdits moyens de liaison coopérant avec lesdits connecteurs internes, les électrodes permettent de réaliser une mesure électrique relative au fluide présent dans la première chambre,
- des moyens de circulation de fluide, lesdits moyens de circulation de fluide étant reliés auxdits moyens d'analyse en coopérant avec au moins un desdits connecteurs et avec lesdits moyens de liaison.

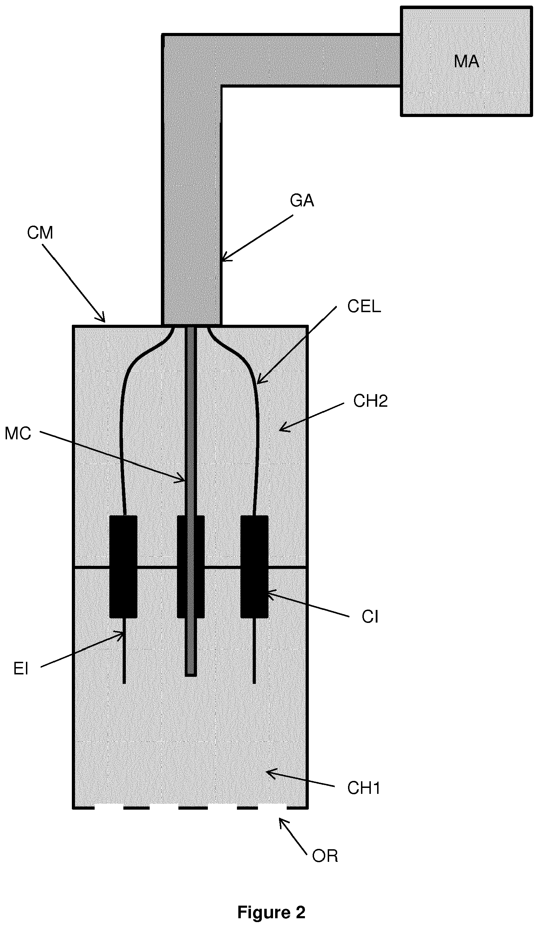

La Figure 1 présente un exemple de réalisation non limitatif du dispositif selon l'invention, les différents éléments du dispositif selon l'invention pouvant être agencés entre eux et dans l'absolu de façon différente. Les Figures 2 à 5 représentent de manière non limitative et non exhaustive d'autres variantes de réalisation du dispositif selon l'invention. Les Figures 1 à 5 correspondent à une coupe selon un plan vertical du dispositif pour la surveillance d'une formation souterraine contenant un fluide.

Le dispositif selon l'invention comporte une cellule de mesure CM, destinée à être disposée dans une cavité formée dans la formation souterraine considérée. La cavité peut avoir été formée par un forage par exemple, à la taille de la cellule de mesure CM, de sorte à ce que la cellule CM soit en contact direct avec la formation. Selon l'invention, la cellule de mesure CM est constituée d'au moins une première chambre CH1 et une deuxième chambre CH2. Avantageusement, ladite deuxième chambre CH2 est agencée au-dessus de ladite première chambre CH1 comme présenté non limitativement en Figure 1.

Selon l'invention, le dispositif est équipé de moyens d'analyse MA qui peuvent être déportés, par exemple en surface, et de moyens de liaison ML reliant de manière étanche ladite cellule de mesure CM aux moyens d'analyse MA. Selon l'invention, les moyens de liaison sont eux-mêmes protégés par des moyens de protection étanches.

Selon l'invention, la paroi de la première chambre CH1 est percée d'une pluralité d'orifices OR permettant le prélèvement du fluide, présent dans la formation, dans le volume interne de la première chambre CH1. Selon l'invention, la deuxième chambre CH2 est quant à elle étanche au fluide. Par ailleurs, selon l'invention, au moins trois connecteurs internes CI relient de manière étanche la première chambre CH1 à la deuxième chambre CH2.

Selon l'invention, la première chambre CH1 comporte en outre au moins deux électrodes internes El, coopérant avec au moins deux des connecteurs internes CI de ladite cellule, les électrodes internes El étant reliées aux moyens d'analyse MA via les moyens de liaison ML, les moyens de liaison ML coopérant eux-mêmes avec les connecteurs internes Cl.

Selon l'invention, la première chambre CH1 comporte en outre des moyens de circulation MC du fluide prélevé dans la première chambre CH1, les moyens de circulation MC du fluide prélevé dans la première chambre CH1 étant reliés auxdits moyens d'analyse MA en coopérant avec au moins un desdits connecteurs CI et avec lesdits moyens de liaison ML.

Selon l'invention, les moyens de liaison assurent une liaison étanche entre la cellule de mesure et les moyens d'analyse, permettant ainsi la transmission sans perte des mesures réalisées par la cellule de mesure aux moyens d'analyse. De plus les moyens de liaison sont protégés par des moyens de protection étanches. Ceci permet de garantir la non altération dans le temps des moyens de liaison eux-mêmes, et ainsi de contribuer à la longévité du dispositif selon l'invention, et à l'adaptation du dispositif selon l'invention à une surveillance à long terme d'une formation souterraine contenant un fluide.

L'étanchéité de la deuxième chambre permet de protéger la partie des éléments du dispositif selon l'invention située dans cette deuxième chambre (au moins les moyens de liaison ML comme montré en Figure 1), et ainsi de contribuer à la non altération dans le temps de la partie des éléments en question, et par là même à l'adaptation du dispositif selon l'invention à une surveillance à long terme d'une formation souterraine contenant un fluide.

L'étanchéité des connecteurs internes vise à prévenir le passage d'une partie du fluide prélevé dans la première chambre vers la deuxième chambre, et ainsi une transmission erronée de la quantité de fluide prélevée aux moyens d'analyse, mais aussi de préserver l'intégrité de la deuxième chambre et de la partie des éléments de mesure du dispositif selon l'invention (au moins les moyens de liaison ML comme montré en Figure 1) qu'elle contient.

Selon un mode de mise en oeuvre de l'invention, les moyens de liaison comportent des moyens d'alimentation électrique des électrodes, et les moyens de protection étanches comportent une gaine étanche protégeant au moins les moyens de circulation de fluide et les moyens d'alimentation électrique. La Figure 2 représente de manière non limitative une variante de ce mode de mise en oeuvre. Pour cette variante, les moyens de protection étanches comportent une gaine étanche GA, reliant le toit de la cellule de mesure CM aux moyens d'analyse MA, et les moyens d'alimentation électrique correspondent à des câbles électriques CEL, les câbles électriques CEL coopérant avec les connecteurs internes Cl, les connecteurs internes CI coopérant avec deux électrodes internes El. Selon ce mode de mise en oeuvre, une première partie des câbles électriques CEL traverse la deuxième chambre CH2, et une deuxième partie (non représentée), insérée dans la gaine GA, relie le toit de la deuxième chambre aux moyens d'analyse MA. Selon ce mode de mise en oeuvre de l'invention, une ouverture est pratiquée dans le toit de la deuxième chambre CH2, cette ouverture coopérant avec la gaine GA. Selon ce même mode de mise en oeuvre de l'invention représenté en Figure 2, le fluide prélevé dans la première chambre CH1 de la cellule de mesure CM est pris en charge par des moyens de circulation de fluide MC, dont une première partie est située dans la première chambre CH1, une deuxième partie traverse au moins un desdits connecteurs internes Cl, une troisième partie traverse la deuxième chambre CH2, et une quatrième partie (non représentée), insérée dans la gaine GA, relie le toit de la deuxième chambre CH2 aux moyens d'analyse MA. Selon un mode de mise en oeuvre de l'invention, les câbles électriques sont isolés par une gaine siliconée et la gaine est constituée en polymère armé par une spirale en acier.

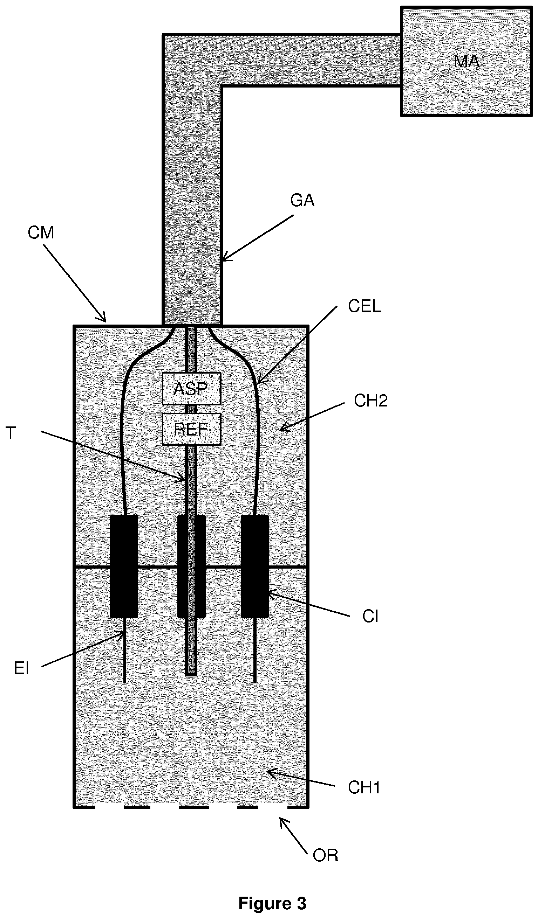

Les Figures 3 à 5, illustratives d'autres modes de réalisation de la présente invention, seront par la suite représentées de manière non limitative et non exhaustive selon des moyens de liaison comprenant une gaine GA, des câbles électriques CEL et des moyens d'analyse MA déportés, la partie des câbles électriques et la partie des moyens de circulation MC situées à l'extérieur de la cellule de mesure CM étant insérées dans la gaine GA. Toute autre constitution et configuration des moyens de liaison permettant de relier la cellule de mesure CM aux moyens d'analyse MA est toutefois possible, les moyens d'analyse MA pouvant être par ailleurs déportés ou non.

Selon un mode de mise en oeuvre de la présente invention, les orifices de la première chambre peuvent être :
(1) recouverts d'une membrane semi-perméable/hydrophobe (par exemple de type polypropylène hydrophobe, de diamètre 0.2 µm), de façon à prévenir le prélèvement d'eau dans la première chambre, et la remontée d'eau dans les moyens de circulation de fluide et vers les moyens d'analyse. Ce mode de mise en oeuvre est en particulier adapté lorsque le fluide d'intérêt est un gaz et dans le cas d'une zone ou l'ennoiement est envisagé (zone inondable, fort battement de la nappe, fortes pluies et mauvaises conditions de drainage) ; ou bien
(2) recouverts d'un géotextile, les géotextiles ayant la propriété d'être hydrophiles (ce qui permet d'assurer un bon couplage hydrique et électrique entre le volume interne de la première chambre et la formation) et anti-particulaires (ce qui permet d'empêcher les particules fines de pénétrer dans le volume interne de la première chambre). Ce mode de mise en oeuvre peut être privilégié dans les zones bien drainées, loin des cours d'eau, hors d'atteinte de la nappe phréatique, et hors risque d'ennoiement ; ou bien
(3) libres, c'est-à-dire non recouverts par un quelconque matériau. Cette dernière mise en oeuvre permet d'avoir une connectivité maximale du volume interne de la première chambre avec le milieu externe, et permet donc de mesurer des fluctuations rapides des compositions de gaz.

Selon un mode particulier de réalisation de la présente invention, la première chambre ainsi que la seconde chambre sont constituées en polytétrafluoroéthylène (PTFE), afin d'assurer une bonne longévité de la cellule de mesure CM ainsi qu'une influence chimique minimale sur l'environnement proche.

Selon un mode de mise en oeuvre non limitatif de la présente invention représenté en Figure 3, les moyens de circulation de fluide comprennent au moins un tube T, un système d'aspiration ASP, et un système de reflux REF. Le système d'aspiration ASP permet d'aspirer le fluide prélevé dans la première chambre CH1, de le faire passer dans au moins un connecteur interne CI puis dans le tube T, et de transférer ainsi ce fluide aux moyens d'analyse MA. Le système de reflux REF permet de refluer le fluide, une fois prélevé puis analysé par les moyens d'analyse MA, vers ladite formation, en repassant par ledit tube T, ledit connecteur interne CI, ladite première chambre CH1, puis lesdits orifices OR de la première chambre CH1. Ce mode de mise en oeuvre permet, en prélevant le fluide et en le réinjectant dans la formation, de fortement limiter la perturbation de l'environnement de mesure, ce qui est approprié pour une surveillance à long terme d'un site contenant un fluide. Préférentiellement, les systèmes d'aspiration ASP et de reflux REF sont déportés, par exemple à la surface de la formation. L'exemple de réalisation de l'invention représenté en Figure 3 comporte des moyens de liaison comprenant une gaine GA, des câbles électriques CEL et des moyens d'analyse MA déportés, la partie des câbles électriques CEL ainsi que la partie du tube T à l'extérieur de la cellule de mesure CM étant insérées dans la gaine GA. Toute autre constitution et configuration des moyens de liaison, et tout autre mode de coopération de ces moyens de liaison avec les moyens de circulation du fluide sont toutefois possibles, les moyens d'analyse pouvant être par ailleurs déportés ou non.

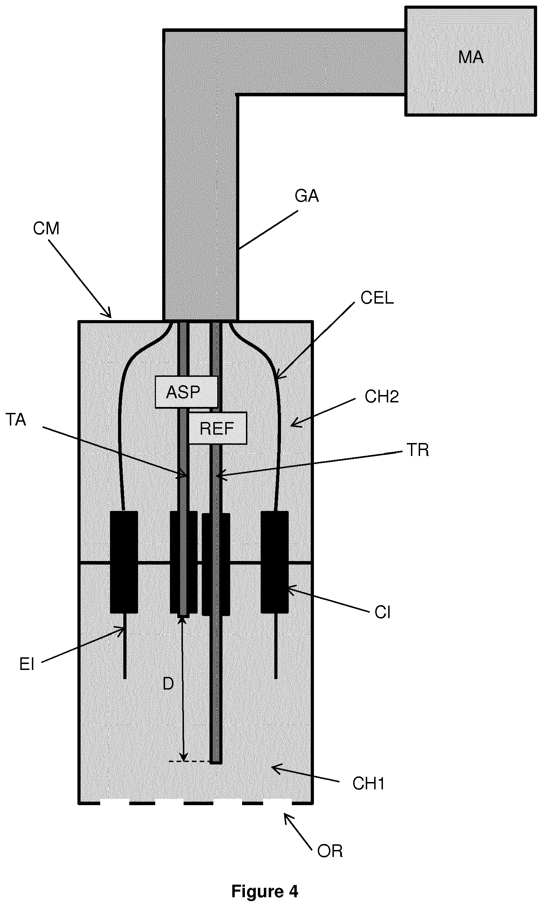

Selon un mode de mise en oeuvre non limitatif de l'invention représenté en Figure 4, les moyens de circulation de fluide comprennent deux tubes TA et TR, le tube TA étant relié au système d'aspiration ASP, et le tube TR étant relié au système de reflux REF. Préférentiellement, le tube TR pénètre profondément à l'intérieur du volume intérieur de la première chambre CH1, alors que la terminaison du tube TA dans la première chambre CH1 correspond sensiblement à la base du connecteur CI avec lequel il coopère. Cette configuration permet de maximiser la distance D entre le point d'aspiration et le point de reflux, et ainsi de maximiser le brassage du gaz entre les deux tubes. Ainsi, les variations de composition du gaz dans la cellule seront plus finement détectables. Préférentiellement, la distance D correspondra à 70% de la hauteur de la première chambre CH1. L'exemple de réalisation de l'invention représenté en Figure 4 comporte des moyens de liaison comprenant une gaine GA, des câbles électriques CEL, des moyens d'analyse MA déportés, la partie des câbles électriques CEL ainsi que les parties des tubes TA et TR à l'extérieur de la cellule de mesure CM étant insérées dans la gaine GA. Toute autre constitution et configuration des moyens de liaison, et tout autre mode de coopération de ces moyens de liaison avec les moyens de circulation du fluide sont toutefois possibles, les moyens d'analyse pouvant être par ailleurs déportés ou non.

Selon un mode de mise en oeuvre de l'invention, les moyens d'analyse comprennent au moins un analyseur de fluide, qui peut être déporté ou non à la surface. L'analyseur de fluide permet la détection et la quantification (estimation de la concentration par exemple) d'au moins un type de fluide. Préférentiellement, l'analyseur de fluide permet au moins la détection et la quantification du fluide injecté dans la formation.

Selon un mode de mise en oeuvre de l'invention, les électrodes internes peuvent être utilisées pour des mesures de résistivité électrique du milieu. Selon ce même mode de mise en oeuvre, au moins une partie du volume interne de la première chambre est rempli avantageusement par un matériau poreux (et préférentiellement perméable au fluide prélevé par les orifices) de référence, de manière à ce qu'au moins une partie des électrodes internes El soient en contact avec ce matériau poreux. Par matériau poreux de référence, on peut entendre un matériau dont les propriétés pétrophysiques et électriques sont connues. Préférentiellement, les propriétés pétrophysiques du milieu poreux de référence sont au moins la porosité et la perméabilité. Préférentiellement, les propriétés électriques du milieu poreux de référence sont au moins la conductivité électrique. Avantageusement, le matériau poreux peut être du sable de quartz. Ainsi le fluide prélevé dans la première chambre pourra se loger dans les pores du matériau poreux, et une mesure de résistivité, via les électrodes internes, en contact avec ce matériau au moins partiellement saturé en fluide, pourra être effectuée.

Très préférentiellement, les moyens d'analyse comprennent au moins un résistivimètre qui peut être ou non déporté à la surface. Les moyens de liaisons peuvent alors comprendre des câbles électriques permettant de relier les connecteurs internes coopérant avec les électrodes internes au résistivimètre.

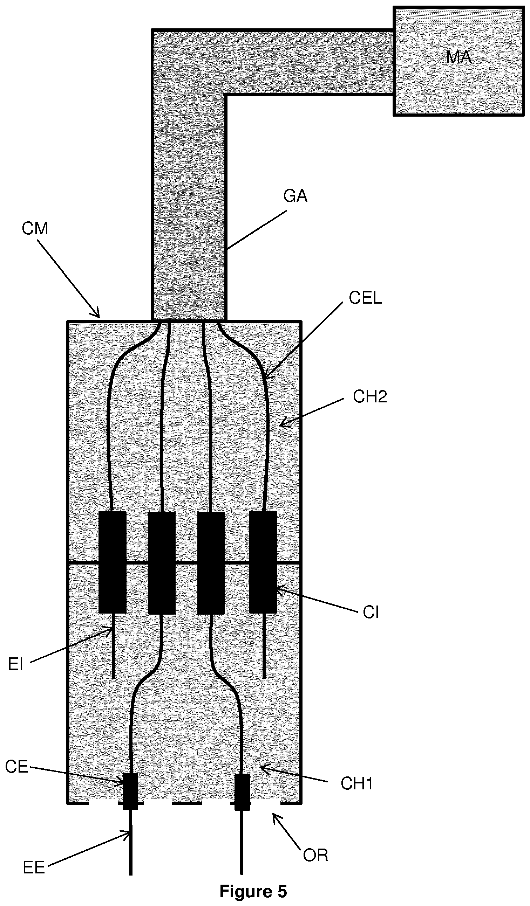

Selon un mode de mise en oeuvre non limitatif de l'invention illustré en Figure 5 (les moyens de circulation de fluide ne sont pas représentés sur cette Figure pour des raisons pures de simplification de la Figure), au moins deux connecteurs externes CE étanches sont placés sur l'une des parois de la première chambre CH1 en contact avec l'extérieur de la cellule de mesure CM, les connecteurs externes CE coopérant avec au moins deux électrodes externes EE. De plus, selon ce même mode de mise en oeuvre, le dispositif comporte en outre au moins deux connecteurs internes CI additionnels, lesdits connecteurs internes CI additionnels permettant le passage de moyens de liaisons (tels que des câbles électriques CEL représentés en Figure 5) entre lesdits connecteurs externes CE et lesdits moyens d'analyse MA. Avantageusement, la paroi sur laquelle sont placés les connecteurs externes CE est en contact avec la formation d'intérêt. Ainsi, les électrodes externes EE traversant cette paroi via les connecteurs externes CE permettent de réaliser des mesures électriques au sein de la formation étudiée. Avantageusement, les moyens d'analyse MA comprennent au moins un résistivimètre et les mesures électriques réalisées au moyen des électrodes externes EE concernent une mesure de la résistivité électrique dans la formation d'intérêt. Selon un mode particulier de mise en oeuvre de la présente invention, on réalise des mesures de résistivité électrique au moyen des au moins deux électrodes internes El et des au moins deux électrodes externes EE placées tel que décrit précédemment. De cette manière, les électrodes internes El permettent de calibrer la mesure de résistivité sur un milieu poreux connu et simple (tel que du sable de quartz), et de servir ainsi de référence par rapport aux mesures de résistivité réalisées par les électrodes externes EE qui seront sujettes à plus d'inconnues vis-à-vis du milieu poreux dans lequel elles sont insérées (composition minéralogique, porosité, granulométrie). Notamment, ce mode de mise en oeuvre peut être utile pour quantifier et surveiller une potentielle dérive des mesures de résistivités liées à une altération des propriétés des électrodes internes et/ou externes. L'exemple de réalisation de l'invention représenté en Figure 5 comporte des moyens de liaison comprenant une gaine GA, des câbles électriques CEL, des moyens d'analyse MA déportés, la partie des câbles électriques CEL à l'extérieur de la cellule de mesure CM étant insérée dans la gaine GA. Toute autre constitution et configuration des moyens de liaison, et toute coopération de ces moyens de liaison avec des moyens de circulation du fluide, est possible, les moyens d'analyse pouvant être par ailleurs déportés ou non. Selon un autre mode de mise en oeuvre de l'invention, les électrodes internes et/ou les électrodes externes sont utilisées pour réaliser des mesures en réflectométrie temporelle (mesures dites TDR, pour « Time Domain Reflectometry » en anglais). Selon ce mode de mise en oeuvre, les moyens d'analyse comprennent un réflectomètre temporel et les électrodes internes et/ou externes sont raccordées via des moyens de liaison (préférentiellement des câbles électriques) au réflectomètre temporel. Un tel mode de mise en oeuvre permet de quantifier la constante diélectrique de la formation étudiée, elle-même proportionnelle à la saturation en eau de la formation étudiée.

Selon un mode de réalisation de la présente invention, le dispositif comporte en outre une sonde de température communiquant avec le volume interne de ladite première chambre via un connecteur interne. Ainsi, le dispositif permet en outre de mesurer la température au sein de la formation étudiée. Cette mesure de la température peut être utile pour déterminer les conductivités thermiques dans la formation étudiée, en comparaison avec une mesure de température de surface, ainsi que pour corriger les mesures de résistivité électrique réalisées par les électrodes internes et/ou externes des effets de la température sur la conductivité du milieu.

Selon un mode de réalisation de la présente invention, le dispositif selon l'invention comporte en outre un moyen de mesure de l'humidité du sol. De telles mesures peuvent en effet permettre de calibrer les mesures électriques réalisées par les électrodes internes et externes vis à vis des variations du taux d'humidité dans le sol.

### Variantes

Des variantes de la présente invention, comportant des éléments permettant des mesures automatisées dans le temps" sont déclinées ci-après. Les variantes décrites ci-dessous peuvent être combinées, seules ou en combinaison, avec l'un quelconque des modes de réalisation décrits précédemment.

Selon un mode de mise en oeuvre de la présente invention, ledit dispositif comprend en outre un automate, permettant de préprogrammer les mesures à réaliser, qu'elles soient de type électrique, géochimique ou de température. L'automate peut par exemple permettre de définir un séquençage des mesures géochimiques, en déclenchant, successivement dans le temps, selon une périodicité donnée, le prélèvement de fluide, ainsi que le transfert et l'analyse de ce fluide. De même, l'automate peut permettre de déclencher des mesures électriques avec une certaine périodicité, selon certains paramètres (nombre d'électrodes impliquées dans la mesure, courant électrique injecté, etc.

Selon un mode de mise en oeuvre de la présente invention, ledit dispositif comprend également un collecteur de données (comme par exemple le modèle DT85GLM commercialisé par la société DIMELCO) et des moyens de transmission des données. Le collecteur de données permet de recueillir les mesures analysées au niveau des moyens d'analyse et de les transmettre, en temps réel, par les moyens de transmission de données.

Selon un mode de réalisation de la présente invention, les moyens de transmission des données collectées sont des moyens de télétransmission (modem permettant une connexion internet par exemple). Préférentiellement, les moyens de transmission des données collectées par le collecteur sont assurés par un modem 3G.

De cette manière, le dispositif selon l'invention permet que les données collectées sur le site soient transmises de façon automatique et en temps réel à un spécialiste, qui pourra ainsi être en mesure de prendre les décisions ad hoc en cas de mesures anormales réalisées in situ par le dispositif.

Selon un mode de réalisation de la présente invention, le collecteur de données permet la prise en compte de seuils déclencheurs d'alerte et est à même de déclencher une alerte. Ainsi, si une quantité de fluide supérieure à un certain seuil fixé par le spécialiste est détectée via l'analyseur de fluide, le collecteur de données est à même de lancer une alerte, par exemple à un spécialiste ou aux pouvoirs publics, via un message électronique, une alerte sonore etc.

Selon un mode de réalisation de la présente invention, l'alimentation électrique du dispositif selon l'invention est assurée par un panneau solaire, et est connectée à une batterie.

Selon un autre mode de réalisation de la présente invention, au moins les moyens d'analyse, ainsi que le collecteur de données et l'automate le cas échéant, sont protégés en surface dans un abri étanche.

Selon un mode de mise en oeuvre dans lequel le dispositif selon l'invention comporte un automate, un collecteur de données, et des moyens de télétransmission des données collectées, le dispositif ainsi constitué peut permettre de réaliser des mesures de manière automatique et préprogrammée via l'automate, d'analyser ces mesures via les moyens d'analyse, de collecter les résultats de ces mesures via le collecteur, puis de les transmettre à un spécialiste situé potentiellement à distance via les moyens de télétransmission. Un dispositif comportant de tels éléments est de fait adapté à une surveillance permanente, à long terme, d'un site contenant un fluide dont l'évolution (chimique et/ou dans l'espace) est à surveiller.

### Utilisation de l'invention

L'invention concerne également l'utilisation du dispositif selon l'invention pour la surveillance d'une formation souterraine contenant un fluide.

L'invention peut plus particulièrement concerner l'utilisation du dispositif selon l'invention pour la surveillance d'un site de stockage géologique de gaz, tel que le dioxyde de carbone (CO₂) ou le méthane.

Préférentiellement, l'utilisation du dispositif selon l'invention pour la surveillance d'une formation souterraine contenant un fluide requiert de réaliser une étape d'étalonnage du dispositif, préalablement à la phase de surveillance proprement dite. Afin de garantir la caractérisation de l'impact sur l'environnement du sol et du sous-sol d'un stockage géologique, une caractérisation préalable à l'activité industrielle en question doit être faite pour définir l'état de référence de l'environnement (aussi appelé « ligne de base »). Les outils de surveillance environnementale doivent donc prévoir la caractérisation du milieu naturel, ses hétérogénéités spatiales et sa variabilité temporelle. Les outils de surveillance doivent ensuite pouvoir être mis en service pour mesurer l'impact d'une activité sur la base d'un état de référence préalablement défini.

Une fois installé sur site, le dispositif selon l'invention peut être exploité en acquisition continue et/ou permanente, ou bien faire l'objet de mesures chroniques. Dans le cas de mesures permanentes, les moyens d'analyse MA pourront être raccordés à la cellule de mesure CM par des moyens de liaison permanents. Dans le cas de mesures chroniques, les moyens de liaison pourront être amovibles et permettre de désolidariser les moyens d'analyse MA de la cellule de mesure CM de façon temporaire, de manière à être en mesure de protéger ces moyens d'analyse MA entre deux mesures.

En outre, l'invention concerne un procédé de surveillance d'une formation souterraine contenant un fluide, au moyen d'un dispositif pour la surveillance d'une formation souterraine contenant un fluide selon l'une quelconque des variantes décrites précédemment, et comprenant au moins les étapes suivantes :
- on fore une cavité destinée à recevoir la cellule de mesure du dispositif ;
- on connecte la cellule de mesure aux moyens de liaison du dispositif ;
- on installe la cellule de mesure au sein de la cavité ainsi formée, et on installe les moyens d'analyse du dispositif en surface ;
- on connecte les moyens d'analyse aux moyens de liaison du dispositif ;
- on réalise des mesures au moyen de ladite cellule de mesure et on analyse lesdites mesures au moyen desdits moyens d'analyse.

### Exemple d'application

Les caractéristiques et avantages du procédé selon l'invention apparaîtront plus clairement à la lecture de l'exemple d'application de l'invention ci-après.

L'exemple d'application en question concerne la surveillance d'une formation souterraine dans laquelle du CO₂ a été injecté, la surveillance étant réalisée par une variante particulière du dispositif selon l'invention illustrée en Figures 6A 6B. Plus précisément la Figure 6A présente une vue externe de la cellule de mesure CM selon le mode de mise en oeuvre de l'invention en question, et la Figure 6B présente une coupe verticale au travers de ladite cellule de mesure CM selon ce même mode de mise en oeuvre. Du fait que la Figure 6B correspond à une section dans le volume de la cellule de mesure CM, l'ensemble des éléments qui constituent le mode de mise en oeuvre en question ne sont pas représentés.

Ainsi, le dispositif tel que mis en oeuvre pour cette application du dispositif selon l'invention est caractérisé par :
- une cellule de mesure CM formée d'un premier corps 01 semi-ouvert, de révolution cylindrique, l'ouverture dudit premier corps étant orientée vers le haut, ledit premier corps étant surmonté par un couvercle 02 de façon à former une première chambre CH1 fermée, le couvercle 02 étant lui-même surmonté d'un deuxième corps semi-ouvert 03, de révolution cylindrique, l'ouverture dudit deuxième corps étant orientée vers le bas, de façon à former, avec ledit couvercle 02 une deuxième chambre CH2 fermée. Ainsi, selon cet exemple de mise en oeuvre, le couvercle 02 constitue à la fois la paroi supérieure de la première chambre CH1 et la paroi inférieure de la deuxième chambre CH2 de la cellule. Selon cet exemple de mise en oeuvre, le premier corps 01, le couvercle 02 et le deuxième corps 03 sont emboités les uns dans les autres de manière étanche via des joints toriques en silicone, puis solidarisés par des goupilles en inox 04 insérées de force dans des orifices prévus à cet effet. Selon ce même mode de mise en oeuvre, la paroi verticale de la première chambre est percée d'orifices OR oblongs et la paroi basale est percée d'orifices OR circulaires, de façon à permettre le passage du gaz présent dans la formation d'intérêt vers le volume interne de la première chambre CH1. Par ailleurs le toit de la deuxième chambre CH2 comporte une ouverture 06 afin de permettre au moins le passage de moyens de liaison ML. Selon ce même mode de mise en oeuvre, les deux corps et le couvercle sont fabriqués en PTFE, afin de garantir une bonne longévité à la cellule de mesure CM. Les chambres ainsi formées ont un diamètre de 100 mm et une hauteur de 100 mm environ chacune.
- cinq orifices percés dans le couvercle 02 de la cellule de mesure CM, le couvercle 02 correspondant à la fois à la paroi supérieure de la première chambre CH1 et à la paroi inférieure de la deuxième chambre CH2, de façon à permettre la communication entre les deux chambres via des connecteurs internes CI ;
- deux orifices percés sur la paroi inférieure de la première chambre CH1, afin de permettre le passage d'électrodes externes EE via des connecteurs externes CE ;
- un volume interne de la première chambre CH1 rempli à 90% par du sable de quartz de granulométrie 300 µm. Ce remplissage permet de laisser au sommet du volume interne de la première chambre CH1 un ciel de gaz d'une hauteur de 2 cm ;
- des parois externes de la cellule de mesure CM (ce qui inclus les orifices percés sur les parois de la première chambre CH1) recouvertes avec un géotextile collé avec une colle à base de silicone. En plus d'assurer un bon couplage hydrique et électrique entre l'intérieur de la première chambre CH1 et la formation, ainsi que d'empêcher le passage de particules dans le volume interne de la première chambre CH1, l'utilisation du géotextile permet en outre de maintenir le sable de quartz à l'intérieur de la première chambre CH1 ;
- quatre électrodes (non représentées) en tungstène, afin de garantir une bonne conductivité électrique et une excellente résistance à la corrosion. Plus particulièrement, les électrodes ont été serties sur des adaptateurs formés d'un côté par un raccord 1/8" de type Swagelok® double bagues, et de l'autre côté par un raccord de type NPT male 1/8". Deux d'entre elles (elles sont alors dites électrodes internes) ont été vissées dans deux des orifices en Téflon® taraudés permettant la communication entre les première et deuxième chambres CH1, CH2. Les deux autres électrodes (elles sont alors dites électrodes externes) ont été vissées dans les deux orifices permettant la communication de la première chambre CH1 avec la formation étudiée. Un connecteur interne Cl ou un connecteur externe CE selon l'invention est donc formé par un raccord 1/8" de type Swagelok® double bagues et un raccord de type NPT male 1/8"°. Les connecteurs internes CI et externes CE associés respectivement à des électrodes internes et à des électrodes externes comprennent des connecteurs électriques en étain permettant le raccordement (par sertissage à la pince) desdites électrodes à câbles électriques coopérant respectivement avec lesdits connecteurs CI, CE. Au moins la partie des câbles électriques située dans la première chambre CH1 et les connecteurs électriques des connecteurs externes CE sont isolés électriquement du volume interne de la première chambre CH1 au moyen d'une gaine thermo-rétractable. Les câbles électriques associés aux électrodes externes remontent vers les moyens d'analyse en passant dans la deuxième chambre CH2 via deux des orifices percés dans le couvercle de la cellule de mesure CM, le passage étant rendu étanche via une colle à base de silicone.
- des moyens de circulation de fluide (non représentés) comprenant un moyen d'aspiration et un moyen de reflux du fluide, chacun comprenant deux tubes, respectivement d'aspiration et de reflux. Les tubes des moyens de circulation de fluide sont des tubes standard 1/8" en inox à bord fin (de diamètre intérieur 1mm (0,04"). Ils sont sertis sur des connecteurs internes CI formés d'un côté par un raccord 1/8" de type Swagelok® double bagues, et de l'autre côté par un raccord de type NPT male 1/8", puis vissés directement dans les orifices en Téflon® taraudés permettant la communication entre les deux chambres. Le tube d'aspiration s'arrête au toit de la première chambre CH1. Le tube de reflux traverse le couvercle et la première chambre CH1, jusqu'à un centimètre au-dessus de la paroi basale de la chambre CH1.
- une sonde de température (non représentée) de type PT100 (commercialisé par la société prosensor), sertie via un connecteur interne CI formé d'un côté par un raccord 1/8" de type Swagelok® double bagues, et de l'autre côté par un raccord de type NPT male 1/8" , vissé directement dans un orifice en Téflon® taraudé permettant la communication entre les deux chambres. Cette sonde de température est traversante et descend jusqu'au milieu de la première chambre CH1. Elle est alimentée par un câble électrique.
- des moyens de liaison (non représentés) comprenant une gaine de protection, armée et flexible, raccordée au toit de la deuxième chambre CH2 par deux écrous de part et d'autre. La gaine tourne librement sur son axe principal, au niveau du raccord au toit de la deuxième chambre CH2, afin de limiter les contraintes sur celle-ci. Les quatre câbles électriques des électrodes, le câble électrique de la sonde de température et les deux tubes des moyens de circulation passent par l'ouverture 06 pratiquée dans le toit de la deuxième chambre CH2 et la gaine avant de rejoindre les moyens d'analyse déportés. La longueur de la gaine est de cinq mètres. Les câbles électriques et tubes ont une longueur de six mètres, permettant une liaison directe avec les moyens d'analyse déportés en surface.
- des moyens d'analyse déportés, qui comprennent un résistivimètre de type Syscal junior 24 (Iris Instruments®), et un analyseur de gaz permettant de détecter et de quantifier le CO2, comme par exemple le détecteur LI-820 commercialisé par la société LI-COR.

Le dispositif selon l'invention tel que précédemment décrit a été installé dans une cavité formée par forage dans la formation étudiée.

Des forages de 100mm ont été réalisés à la tarière manuelle et les échantillons de sol ont été récupérés soigneusement pour chaque intervalle de profondeur de 10 cm. Trois forages ont été réalisés à des profondeurs de: (1) 180 cm (2) 120 cm (3) 60 cm. Les échantillons de sol sont analysés en laboratoire pour définir leur teneur en carbone organique total, en carbone minéral, la répartition en minéraux majeurs (argiles, tecto-silicates et carbonates), la porosité, la distribution granulométrique.

Une fraction du sol correspondant à la base du forage est tamisée grossièrement afin d'en retirer les pierres et les racines. Ce sol tamisé est introduit au fond du forage destiné à recevoir le dispositif selon l'invention afin de remplir de nouveau les 20 derniers centimètres restants. La cellule de mesure CM est descendue manuellement en la poussant via la gaine armée GA, ainsi qu'en guidant la verticalité de la cellule de mesure CM grâce à une perche. Une fois le contact effectué avec la base du forage, la cellule de mesure CM est enfoncée grâce à la perche, en s'assurant que les électrodes externes EE pénètrent bien dans la base du forage par un contrôle de la distance d'enfoncement. Une fois la cellule de mesure CM en place, le forage est comblé de sable (de 100µm de diamètre) jusqu'à recouvrir la cellule, et ce, afin de garantir un bon couplage mécanique des milieux poreux entre l'intérieur de la cellule de mesure CM et la formation étudiée. Le forage est ensuite inondé d'eau douce afin de remanier le milieu poreux, de combler tout espace laissé non rempli, et de maximiser le couplage hydrique entre la cellule de mesure CM et la formation étudiée. Le forage est finalement comblé avec le sol prélevé en respectant sa zonation verticale. Puis, les terminaisons des tubes TA, TR et des câbles CEL sont connectées aux moyens d'analyse MA placés à la surface de l'installation.

Une étape de calibration du dispositif selon l'invention a été préalablement réalisée à l'étape de surveillance à proprement parler. Plus précisément :
- des mesures de résistivité électrique sont réalisées via un test de drainage, en saturant le forage destiné à recevoir la cellule de mesure CM avec de l'eau douce, et en mesurant l'évolution des résistivités, lors de son asséchement par drainage naturel et par évapotranspiration. Des mesures comparables à celles acquises en laboratoire doivent être obtenues. La Figure 7A (respectivement la Figure 7B) présente par exemple des courbes de calibration de la résistivité R mesurée par les électrodes internes El (respectivement par les électrodes externes EE) en fonction de la saturation en eau SW pour trois différentes configurations d'assemblage de la cellule de mesure CM ;
- des mesures de température via la sonde de température sont contrôlées, en comparaison de températures relevées en surface de l'installation du dispositif selon l'invention ;
- la validation des moyens de circulation de fluide est effectuée en injectant un gaz de référence dans le tube de reflux TR, jusqu'à obtenir une mesure de sa composition de référence dans le tube d'aspiration TA. Ensuite l'injection est arrêtée et les moyens de circulation de fluide sont remis en circulation dans une boucle fermée. Les vitesses d'échange mesurées doivent correspondre à celles mesurées en laboratoire pour des saturations en eau équivalente (indiquées par les résistivités).

Les mesures réalisées au moyen du dispositif ainsi implanté dans la formation et ainsi calibré consiste en des mesures de résistivité, des mesures de température et en des analyses de gaz. Le système est installé de manière permanente et des mesures sont réalisées toutes les minutes au moment de l'injection du gaz, et toutes les 30 minutes après l'injection.

Un tel dispositif, constitué d'éléments étanches aux fluides et comprenant des moyens de circulation de fluide permettant de refluer le fluide prélevé dans la formation, est adapté à une surveillance à long terme d'une formation, qui plus est sans perturbation de la mesure. Par ailleurs, la possibilité de réaliser, en une unique cellule de mesure, différents types de mesures physiques (électriques, géochimiques, de température) permet d'éviter de multiplier les installations sur site, et ainsi de réduire les coûts d'installation et la perturbation de l'environnement de mesure. Des mesures de différents types, réalisées simultanément et en un unique et même point de mesure, permettent qui plus est une interprétation croisée plus fiable des différents types de mesures physiques.

## Revendications

1. Dispositif pour la surveillance d'une formation souterraine contenant un fluide, ledit dispositif comportant au moins une cellule de mesure (CM), destinée à être disposée dans une cavité formée dans ladite formation souterraine, des moyens d'analyse (MA) destinés à être en surface, des moyens de liaison (ML) reliant ladite cellule de mesure (CM) auxdits moyens d'analyse (MA), **caractérisé en ce que** :
- ladite cellule (CM) comprend au moins une première chambre (CH1), une deuxième chambre (CH2) étanche audit fluide, et au moins trois connecteurs internes (CI) reliant de manière étanche ladite première chambre (CH1) à ladite deuxième chambre (CH2),
- lesdits moyens de liaison (ML) relient de manière étanche ladite cellule de mesure (CM) auxdits moyens d'analyse (MA), et lesdits moyens de liaison comportent des moyens de protection étanches ;
- ladite première chambre (CH1) comporte au moins :
- une pluralité d'orifices (OR) permettant le passage dudit fluide dans ladite première chambre (CH1),
- au moins deux électrodes internes (EI) coopérant avec au moins deux desdits connecteurs internes (CI), lesdites électrodes internes (EI) étant reliées auxdits moyens d'analyse (MA) via lesdits moyens de liaison (ML), lesdits moyens de liaison (ML) coopérant avec lesdits connecteurs internes (CI),
- des moyens de circulation de fluide (MC), lesdits moyens de circulation de fluide (MC) étant reliés auxdits moyens d'analyse (MA) en coopérant avec au moins un desdits connecteurs (CI) et avec lesdits moyens de liaison (ML).

2. Dispositif selon la revendication 1, dans lequel ledit dispositif comporte en outre au moins deux électrodes externes (EE), deux connecteurs internes (CI) reliant lesdites deux chambres (CH1,CH2), deux connecteurs externes (CE) étanches placés sur au moins une paroi de ladite première chambre, ladite paroi étant en contact avec ladite formation, lesdites électrodes externes (EE) coopérant avec lesdits connecteurs externes (CE), lesdites électrodes externes (EE) étant reliées auxdits moyens d'analyse (MA) via lesdits moyens de liaison (ML), lesdits moyens de liaison (ML) coopérant avec lesdits deux connecteurs internes (CI) et lesdits deux connecteurs externes (CE).

3. Dispositif selon l'une des revendications précédentes, dans lequel lesdits moyens de liaison (ML) comportent des moyens d'alimentation électrique (CEL) desdites électrodes (EI, EE), et lesdits moyens de protection étanches comporte une gaine étanche (GA) protégeant au moins lesdits moyens de circulation (MC) et lesdits moyens d'alimentation électrique (CEL).

4. Dispositif selon l'une des revendications précédentes, dans lequel ladite première chambre (CH1) est remplie par un matériau poreux et perméable pour lequel on connaît des propriétés pétrophysiques et électriques.

5. Dispositif selon la revendication 4, dans lequel lesdites propriétés pétrophysiques sont au moins la porosité et la perméabilité, et lesdites propriétés électriques sont au moins la conductivité électrique.

6. Dispositif selon l'une des revendications précédentes, dans lequel lesdits moyens de liaison (ML), lesdites chambres (CH1, CH2), et lesdits connecteurs (CE, CI) sont fabriqués en PTFE.

7. Dispositif selon l'une des revendications précédentes, dans lequel lesdits moyens d'analyse (MA) comportent au moins un analyseur de fluide et/ou un résistivimètre.

8. Dispositif selon l'une des revendications précédentes, dans lequel lesdits moyens de circulation (MC) de fluide comprennent au moins un tuyau (T), un système d'aspiration de fluide (ASP), et un système de reflux de fluide (REF).

9. Dispositif selon l'une des revendications précédentes, dans lequel lesdits moyens de circulation (MC) de fluide comprennent au moins deux tuyaux (TA, TR), un système d'aspiration de fluide (ASP), et un système de reflux de fluide (REF).

10. Utilisation du dispositif selon l'une des revendications précédentes pour la surveillance d'un site de stockage géologique d'un gaz, tel que du CO₂ ou du méthane.

11. Utilisation selon la revendication 10, dans laquelle une étape d'étalonnage est réalisée préalablement à l'injection de gaz dans ledit site de stockage géologique.

12. Procédé de surveillance d'une formation souterraine contenant un fluide, dans lequel on utilise au moins un dispositif pour la surveillance d'une formation souterraine contenant un fluide selon l'une des revendications 1 à 9, et dans lequel on réalise au moins les étapes suivantes :
- on fore une cavité destinée à recevoir ladite cellule de mesure (CM) dudit dispositif ;
- on connecte ladite cellule de mesure (CM) aux moyens de liaison (ML) dudit dispositif ;
- on installe ladite cellule de mesure (CM) au sein de ladite cavité ainsi formée, et on installe lesdits moyens d'analyse (MA) dudit dispositif en surface ;
- on connecte lesdits moyens d'analyse (MA) auxdits moyens de liaison (ML) dudit dispositif ;
- on réalise des mesures au moyen de ladite cellule de mesure (CM) et on analyse lesdites mesures au moyen desdits moyens d'analyse (MA).

## Patentansprüche

1. Vorrichtung zum Überwachen einer unterirdischen Formation, die ein Fluid enthält, wobei die Vorrichtung mindestens eine Messzelle (CM) aufweist, die zur Anordnung in einem Hohlraum bestimmt ist, der in der unterirdischen Formation ausgebildet ist, Analyseeinrichtungen (MA), die für die Oberfläche bestimmt sind, Verbindungseinrichtungen (ML), die die Messzelle (CM) mit den Analyseeinrichtungen (MA) verbinden, **dadurch gekennzeichnet, dass**:
- die Zelle (CM) mindestens eine erste Kammer (CH1), eine zweite Kammer (CH2), die für das Fluid undurchlässig ist, und mindestens drei interne Verbinder (CI) aufweist, die die erste Kammer (CH1) mit der zweiten Kammer (CH2) auf dichte Weise verbinden,
- wobei die Verbindungseinrichtungen (ML) die Messzelle (CM) mit den Analyseeinrichtungen (MA) auf dichte Weise verbunden, und wobei die Verbindungseinrichtungen dichte Schutzeinrichtungen aufweisen;
- wobei die erste Kammer (CH1) mindestens aufweist:
- mehrere Öffnungen (OR), die den Durchgang des Fluids in die erste Kammer (CF1) ermöglichen,
- mindestens zwei interne Elektroden (E1), die mit mindestens zwei der internen Verbinder (CI) zusammenwirken, wobei die internen Elektroden (E1) mit den Analyseeinrichturigen (MA) über die Verbindungseinrichtungen (ML) verbunden sind, wobei die Verbindungseinrichtungen (ML) mit den internen Verbindern (CI) zusammenwirken,
- Fluidzirkulationseinrichtungen (MC), wobei die Fluidzirkulationseinrichtungen (MC) mit Analyseeinrichtungen (MA) verbunden sind, indem sie mit mindestens einem der Verbinder (CI) und mit den Verbindungseinrichtungen (ML) zusammenwirken.

2. Vorrichtung nach Anspruch 1, wobei die Vorrichtung ferner mindestens zwei externe Elektroden (EE), zwei interne Verbinder (CI), die die beiden Kammern (CH1, CH2) verbinden, zwei dichte externe Verbinder (CE) aufweist, die auf mindestens einer Wand der ersten Kammer angeordnet sind, wobei die Wand mit der Formation in Kontakt ist, wobei die externen Elektroden (EE) mit den externen Verbindern (CE) zusammenwirken, wobei die externen Elektroden (EE) mit den Analyseeinrichtungen (MA) über die Verbindungseinrichtungen (ML) verbunden sind, wobei die Verbindungseinrichtungen (ML) mit den beiden internen Verbindern (CI) und den beiden externen Verbindern (CE) zusammenwirken.

3. Vorrichtung nach einem der vorhergehenden Ansprüche, wobei die Verbindungseinrichtungen (ML) Stromversorgungseinrichtungen (CEL) der Elektroden (E1, EE) umfassen, und die dichten Schutzeinrichtungen eine dichte Hülle (GA) umfassen, die mindestens die Zirkulationseinrichtungen (MC) und die Stromversorgungseinrichtungen (CEL) schützt.

4. Verfahren nach einem der vorhergehenden Ansprüche, wobei die erste Kammer (CH1) mit einem porösen und durchlässige Material gefüllt ist, dessen petrophysikalischen und elektrischen Eigenschaften bekannt sind.

5. Vorrichtung nach Anspruch 4, wobei die petrophysikalischen Eigenschaften mindestens die Porosität und die Durchlässigkeit sind, und die elektrischen Eigenschaften mindestens die elektrische Leitfähigkeit sind.

6. Vorrichtung nach einem der vorhergehenden Ansprüche, wobei die Verbindungseinrichtungen (ML), die Kammern (CH1, CH2) und die Verbinder (CE, CI) aus PTFE hergestellt sind.

7. Vorrichtung nach einem der vorhergehenden Ansprüche, wobei die Analyseeinrichtungen (MA) mindestens einen Fluid-Analysator und/oder einen Resistivitätsmesser umfassen.

8. Vorrichtung nach einem der vorhergehenden Ansprüche, wobei die Fluidzirkulationseinrichtungen (MC) mindestens ein Rohr (T), ein Fluidansaugsystem (ASP) und ein Fluidrückführungssystem (REF) umfassen.

9. Vorrichtung nach einem der vorhergehenden Ansprüche, wobei die Fluidzirkulationseinrichtungen (MC) mindestens zwei Rohre (TA, TR), ein Fluidansaugsystem (ASP) und ein Fluidrückführungssystem (REF) umfassen.

10. Verwendung der Vorrichtung nach einem der vorhergehenden Ansprüche zur Überwachung einer geologischen Lagerstätte eines Gases, wie beispielsweise CO₂ oder Methan.

11. Verwendung nach Anspruch 10, wobei ein Kalibrierschritt vor der Injektion von Gas in die geologische Lagerstätte ausgeführt wird.

12. Verfahren zum Überwachen einer unterirdischen Formation, die ein Fluid enthält, wobei mindestens eine Vorrichtung zur Überwachung einer unterirdischen Formation verwendet wird, die ein Fluid nach einem der Ansprüche 1 bis 9 enthält, und wobei mindestens die folgenden Schritte durchgeführt werden:
- Bohren eines Hohlraums, der dazu bestimmt ist, die Messzelle (CM) der Vorrichtung aufzunehmen;
- Verbinden der Messzelle (CM) mit den Verbindungseinrichtungen (ML) der Vorrichtung;
- Anordnen der Messzelle (CM) innerhalb des auf diese Weise gebildeten Hohlraums, und Anordnen der Analyseeinrichtungen (MA) der Vorrichtung an der Oberfläche;
- Verbinden der Analyseeinrichtungen (MA) mit den Verbindungseinrichtungen (ML) der Vorrichtung;
- Ausführen der Messungen mithilfe der Messzelle (CM) und Analysieren der Messungen mithilfe der Analyseeinrichtungen (MA).

## Claims

1. A device for monitoring an underground formation containing a fluid, said device comprising at least one measuring cell (CM) intended to be arranged in a cavity provided in said underground formation, analysis means (MA) intended to be arranged on the surface, connection means (ML) connecting said measuring cell (CM) to said analysis means (MA), **characterized in that**:
- said cell (CM) comprises at least a first chamber (CH1), a second chamber (CH2) impervious to said fluid, and at least three inner connectors (CI) sealingly connecting said first chamber (CH1) to said second chamber (CH2),
- said connection means (ML) sealingly connect said measuring cell (CM) to said analysis means (MA), and said connection means comprise sealed protection means,
- said first chamber (CH1) comprises at least:
- a plurality of orifices (OR) allowing passage of said fluid into said first chamber (CH1),
- at least two inner electrodes (EI) cooperating with at least two of said inner connectors (CI), said inner electrodes (EI) being connected to said analysis means (MA) through said connection means (ML), said connection means (ML) cooperating with said inner connectors (CI),
- fluid circulation means (MC), said fluid circulation means (MC) being connected to said analysis means (MA) while cooperating with at least one of said connectors (CI) and with said connection means (ML).

2. A device as claimed in claim 1, wherein said device further comprises at least two outer electrodes (EE), two inner connectors (CI) connecting said two chambers (CH1, CH2), two sealed outer connectors (CE) arranged on at least one wall of said first chamber, said wall being in contact with said formation, said outer electrodes (EE) cooperating with said outer connectors (CE), said outer electrodes (EE) being connected to said analysis means (MA) through said connection means (ML), said connection means (ML) cooperating with said two inner connectors (CI) and said two outer connectors (CE).

3. A device as claimed in any one of the previous claims, wherein said connection means (ML) comprise power supply means (CEL) for said electrodes (EI, EE), and said sealed protection means comprise a sealed sheath (GA) protecting at least said circulation means (MC) and said power supply means (CEL).

4. A device as claimed in any one of the previous claims, wherein said first chamber (CH1) is filled with a permeable porous material for which petrophysical and electrical properties are known.

5. A device as claimed in claim 4, wherein said petrophysical properties are at least porosity and permeability, and said electrical properties are at least electrical conductivity.

6. A device as claimed in any one of the previous claims, wherein said connection means (ML), said chambers (CH1, CH2) and said connectors (CE, CI) are made of PTFE.

7. A device as claimed in any one of the previous claims, wherein said analysis means (MA) comprise at least a fluid analyzer and/or a resistivity meter.

8. A device as claimed in any one of the previous claims, wherein said fluid circulation means (MC) comprise at least a pipe (T), a fluid suction system (ASP) and a fluid reflux system (REF).

9. A device as claimed in any one of the previous claims, wherein said fluid circulation means (MC) comprise at least two pipes (TA, TR), a fluid suction system (ASP) and a fluid reflux system (REF).

10. Use of the device as claimed in any one of the previous claims for monitoring a geological storage site for a gas such as CO₂ or methane.

11. Use as claimed in claim 10, wherein a calibration stage is carried out prior to injecting gas into said geological storage site.

12. A method for monitoring an underground formation containing a fluid, wherein at least one device for monitoring an underground formation containing a fluid as claimed in any one of claims 1 to 9 is used and wherein at least the following steps are carried out:
- drilling a cavity intended to receive said measuring cell (CM) of said device,
- connecting said measuring cell (CM) to connection means (ML) of said device,
- arranging said measuring cell (CM) within said cavity thus formed and arranging said analysis means (MA) of said device on the surface,
- connecting said analysis means (MA) to said connection means (ML) of said device,
- performing measurements using said measuring cell (CM) and analysing said measurements using said analysis means (MA).
